# EUROPEAN PATENT APPLICATION

(11) **EP 4 775 154 A1**
(43) Date of publication of application: **15.07.2026**
(21) Application number: 24871849.6
(22) Date of filing: 11.09.2024
(51) Int. Cl.: A61B 17/02, A61B 18/14

(54) **METHOD FOR MANUFACTURING EXPANDABLE BODY, METHOD FOR MANUFACTURING EXPANDABLE BODY ASSEMBLY, AND MEDICAL DEVICE**

(30) Priority: 29.09.2023 JP 2023169566
(71) Applicant: Terumo Kabushiki Kaisha, Tokyo 151-0072 (JP)
(72) Inventor: TAKAHASHI, Yusuke, Ashigarakami-gun, Kanagawa 259-0151 (JP)
(74) Representative: Prüfer & Partner mbB Patentanwälte · Rechtsanwälte
(86) International application number: PCT/JP2024/032489
(87) International publication number: WO 2025/070052

(57) **Abstract**

The present invention provides a method for manufacturing an expansion body, a method for manufacturing an expansion body assembly, and a medical device, with which it is possible to inhibit breakage of an expansion body during manufacture.

Provided is a method for manufacturing an expansion body (21) that includes a plurality of struts (50) extending along the axial direction and aligned in the circumferential direction around the axial direction, and has, in the middle in the axial direction, a waist portion (55) having a reduced outer diameter, the method including expanding an inside of a base material (180) having a tubular shape and including the plurality of struts (50) using an inner mold, and restricting an outside of the base material (180) with an outer mold (150) in order to form a bottom (56) having the smallest outer diameter of the waist portion (55) and an upright portion extending radially outward from the bottom (56).

## Description

### Technical Field

The present invention relates to a method for manufacturing an expansion body, a method for manufacturing an expansion body assembly, and a medical device.

### Background Art

In order to expand a biological lumen or a through hole of a biological membrane for medical use, an expansion body made of a shape memory alloy, insertable into a living body in a contracted state, and expandable in the living body is used (see, for example, Patent Literature 1).

In general, in order to allow the expansion body made of a shape memory alloy to remember a shape, a heat treatment is applied in a state where the expansion body is shaped using a mold. An expansion body used for expanding a biological lumen or a through hole of a biological membrane for medical use is manufactured by inserting an inner mold into a tube material processed by a laser processing machine or the like or a mesh-like tube body formed by interweaving a plurality of wire materials to expand the tube material or the tube body from a tube shape.

### Citation List

### Patent Literature

Patent Literature 1: WO 2019-085841 A

### Summary of Invention

### Technical Problem

In a case where the expansion body to be obtained has a waist portion that is constricted with a sharply reduced outer diameter, the expansion body may have increased strain if it is attempted to be shaped by one deformation, and thus, a risk of breakage increases.

The present invention has been made to solve the above-described problems, and an object of the present invention is to provide a method for manufacturing an expansion body, a method for manufacturing an expansion body assembly, and a medical device with which it is possible to inhibit breakage of an expansion body during manufacture.

### Solution to Problem

(1) The method for manufacturing an expansion body according to the present invention for achieving the above object is a method for manufacturing an expansion body that includes a plurality of struts extending along an axial direction and aligned in a circumferential direction around the axial direction, and has, in the middle in the axial direction, a waist portion having a reduced outer diameter, the method including expanding an inside of a base material that has a tubular shape and includes the plurality of struts using an inner mold, and restricting an outside of the base material with an outer mold in order to form a bottom having the smallest outer diameter of the waist portion and an upright portion extending radially outward from the bottom.

### Advantageous Effects of Invention

In (1) the method for manufacturing the expansion body, the bottom and the upright portion of the waist portion are formed using the inner mold and the outer mold, and thus, the method can reduce the strain generated in the expansion body due to the rapid deformation during manufacture and inhibit the breakage of the expansion body.

(2) In the method for manufacturing an expansion body according to (1), the inner mold may include a first inner mold, and the method may include a first step of forming a half side positioned on one side in the axial direction from the bottom of the expansion body together with the bottom using the first inner mold and the outer mold, and a second step of forming another half side positioned on a side opposite to the one side of the expansion body. With this configuration, in the method for manufacturing an expansion body, the two half sides are formed in separate steps, and thus, the method can reduce the strain generated in the expansion body and inhibit the breakage of the expansion body.

(3) In the method for manufacturing an expansion body according to (2), the outside of the base material into which the first inner mold has been inserted may be covered with the outer mold that is not a split mold and that has a ring shape in the first step and the second step. When the outer mold is a split mold, each piece of the split mold may deform differently due to repeated use. On the other hand, the outer mold has a ring shape, and thus, deformation of the outer mold due to repeated use can be reduced, whereby the expansion body having an appropriate shape can be manufactured.

(4) In the method for manufacturing an expansion body according to (2) or (3), the first inner mold and the outer mold may be commonly used in the first step and the second step to form both of the half sides located on both sides from the bottom in the axial direction. With this configuration, the two half sides located across the bottom can be formed in the same shape.

(5) In the method for manufacturing an expansion body according to any one of (2) to (4), an inside of the half side formed in the first step may be supported with a support inner mold including a projecting portion projecting radially outward in the second step. With this configuration, in the second step, deformation of the shape of the half side formed in the first step can be reduced, so that the shape of the half side formed in the first step can be appropriately maintained.

(6) In the method for manufacturing an expansion body according to any one of (2) to (5), a vicinity of the bottom inside the other half side to be formed in the second step may be expanded in the first step using a second inner mold including an outer diameter constant portion that continuously has an outer diameter substantially equal to an outer diameter of the bottom in the axial direction. With this configuration, when the half side on one side is formed in the first step, the other half side can be deformed to the same extent as the bottom. As a result, it is possible to reduce the strain generated in the expansion body in the second step and to inhibit the breakage of the expansion body.

(7) In the method for manufacturing an expansion body according to any one of (2) to (6), a vicinity of the bottom inside the other half side to be formed in the second step may be expanded in the first step using a second inner mold including an outer diameter constant portion that continuously has an outer diameter substantially equal to an outer diameter of the bottom in the axial direction, and in the second step, the other half side may be formed using the first inner mold and the outer mold and an inside of the half side formed in the first step may be supported with a support inner mold including a projecting portion projecting radially outward. With this configuration, the first inner mold and the outer mold can be commonly used in the first step and the second step, whereby the two sides located across the bottom can be easily formed in the same shape.

(8) The method for manufacturing an expansion body according to any one of (2) to (7) may further include expanding the base material into a spindle shape using a preform mold having a maximum outer diameter smaller than an inner diameter of the bottom of the first inner mold before the first step. With this configuration, the base material can be deformed to some extent before the first step. As a result, it is possible to reduce the strain generated in the expansion body in the first step and to inhibit the breakage of the expansion body.

(9) The method for manufacturing an expansion body according to any one of (2) to (8) may further include a step of forming a plurality of first slits along the axial direction in a first end in the axial direction of the base material before the first step, and in the first step and the second step, the first end is widened and the inner mold is inserted into the base material from the first end. With this configuration, the first end is easily widened, whereby it is easy to insert the inner mold from the first end into the base material and to place the inner mold at a desired position.

(10) The method for manufacturing an expansion body according to (9) may further include disconnecting, from the base material, a ring-shaped connection portion that has been placed at a second end on a side opposite to the first end of the base material for maintaining a tubular shape of the second end, after the second step. With this configuration, the method for manufacturing the expansion body can easily and firmly fix the second end to the distal part of the shaft portion.

(11) The method for manufacturing an expansion body according to (9) or (10) may further include a step of forming a second slit in the second end along the axial direction and forming a restricting portion for restricting widening of the second slit. With this configuration, the method for manufacturing an expansion body facilitates the insertion of the shaft portion to be connected into the second end by releasing the restricting portion. Further, after the shaft portion is inserted into the second end, the restricting portion restricts the expansion of the second slit, whereby the second end can be easily fixed to the shaft portion.

(12) A method for manufacturing an expansion body assembly according to the present invention for achieving the above object includes: cutting out a base material having a tubular shape to form a plurality of struts extending in an axial direction of the base material and aligned in a circumferential direction around the axial direction and to form, at a second end on a side opposite to a first end in the axial direction of the base material, a fixing portion having a tubular shape and including a second slit along the axial direction and a restricting portion for restricting widening of the second slit, and a ring-shaped connection portion connected to the fixing portion and maintaining the tubular shape of the fixing portion; deforming the plurality of struts of the base material in a radial direction using a mold to form an expansion body with a waist portion having a reduced outer diameter in a middle in the axial direction; disconnecting the ring-shaped connection portion from the fixing portion and widening the second slit by removing restriction by the restricting portion after forming the expansion body; inserting a shaft portion that is long into the expansion body; and after covering a distal part of the shaft portion with the second end, restricting widening of the second slit by the restricting portion and fixing the second end to the distal part of the shaft portion. With this configuration, in the method for manufacturing an expansion body assembly, the tubular shape of the fixing portion which is more likely to be widened by the second slit can be suppressed by the ring-shaped connection portion, until the expansion body is created from the base material and the expansion body is fixed to the shaft portion. Therefore, the expansion body and the expansion body assembly can be easily manufactured. In addition, the ring-shaped connection portion is disconnected after the expansion body is formed, and thus, the second slit can be widened. Accordingly, it is easy to insert the shaft portion into the second end and to fix the second end to the shaft portion by means of the restricting portion.

(13) A medical device according to the present invention for achieving the above object includes: an expansion body including a plurality of struts extending along an axial direction and aligned in a circumferential direction around the axial direction; and a shaft portion that is long and that is connected to a proximal part of the expansion body, wherein the proximal part of the expansion body includes a fixing portion having a tubular shape, provided with at least one proximal slit along the axial direction, and capable of opening and closing the proximal slit, one of two edges of the fixing portion located across the proximal slit is formed with a protrusion protruding to another edge in the circumferential direction, while the other edge is provided with a recess recessed to be engaged with the protrusion, the proximal slit being formed to include a boundary between the protrusion and the recess, the protrusion includes a base formed at one end in the circumferential direction, and a top formed on another end in the circumferential direction and longer than the base in the axial direction of the expansion body, the recess includes an opening top that receives the base of the protrusion and an opening bottom that communicates with the opening top and receives the top of the protrusion, the top has a length in the circumferential direction shorter than a length in the circumferential direction of the opening bottom, and the proximal part of the expansion body is fixed to the shaft portion in a state in which the protrusion is fitted into the recess and a gap in the circumferential direction is formed between the top of the protrusion and the opening bottom of the recess. With this configuration, in the medical device, the top of the protrusion of the expansion body is engaged with the opening bottom of the recess to fix the expansion body to the shaft portion. In addition, since the gap in the circumferential direction is formed between the top of the protrusion and the opening bottom of the recess, the second end can be satisfactorily fixed to the shaft portion even if the outer diameter of the shaft portion varies.

(14) In the medical device according to (13), the fixing portion may have, at a proximal end, a cut mark obtained by cutting the ring-shaped connection portion. With this configuration, the medical device can maintain the tubular shape of the fixing portion by the ring-shaped connection portion until necessary at the time of manufacturing, and thus is easy to manufacture.

(15) In the medical device according to (14), the proximal end of the fixing portion may include a depressed portion that is recessed toward a distal end of the expansion body and is provided with the cut mark inside. With this configuration, it is possible to prevent the medical device from damaging another medical device or biological tissue due to the cut mark contacting therewith.

(16) In the medical device according to any one of (13) to (15), the plurality of struts may include a plurality of strut groups extending toward the proximal part of the expansion body, the fixing portion may include at least one separation portion separated in the circumferential direction by the proximal slit, and the at least one separation portion may be each connected to a proximal end of a corresponding one of the strut groups. With this configuration, in the medical device, the separation portion is formed in accordance with the strut groups, whereby it is possible to prevent the occurrence of distortion of the shape of the expansion body due to the excessive number of separation portions.

(17) In the medical device according to any one of (13) to (16), distal parts of the plurality of struts may include a plurality of distal struts that extends in the axial direction and is independent from each other in the circumferential direction, and the distal part of the expansion body may include a distal shaft portion that fixes the plurality of distal struts in such a manner that two adjacent distal struts are not separated from each other in the circumferential direction. With this configuration, in the medical device, the plurality of independent distal struts is fixed to the distal shaft portion, whereby it is possible to prevent the occurrence of distortion of the shape of the distal part of the expansion body.

### Brief Description of Drawings

[Fig. 1] Fig. 1 is a side view illustrating a medical device including an expansion body.
[Fig. 2] Fig. 2 is a side view illustrating a distal part of the medical device.
[Fig. 3] Fig. 3 is a perspective view illustrating a mold together with a core member and a fixture.
[Fig. 4] Fig. 4 is a cross-sectional view illustrating the mold.
[Fig. 5] Fig. 5 is a cross-sectional view illustrating a first use example of the mold.
[Fig. 6] Fig. 6 is a cross-sectional view illustrating a second use example of the mold.
[Fig. 7] Fig. 7 is a side view illustrating a base material.
[Fig. 8] Fig. 8 is a side view illustrating a state in which a preform mold is inserted into the base material.
[Fig. 9] Fig. 9 is a side view illustrating a state in which a first inner mold and a second inner mold are inserted into the base material.
[Fig. 10] Fig. 10 is a side view illustrating a state in which the base material into which the first inner mold and the second inner mold have been inserted is covered with an outer mold.
[Fig. 11] Fig. 11 is a side view illustrating the base material from which the mold is removed.
[Fig. 12] Fig. 12 is a side view illustrating a state in which the base material into which the first inner mold has been inserted is covered with the outer mold, and a support inner mold is further inserted into the base material.
[Fig. 13] Fig. 13 is a side view illustrating a manufactured expansion body.
[Fig. 14] Fig. 14 is a side view illustrating a second end of the expansion body, in which (A) illustrates a state in which restriction by a restricting portion is removed, and (B) illustrates a state in which the restriction by the restricting portion is applied.
[Fig. 15] Fig. 15 is a side view illustrating an expansion body assembly.
[Fig. 16] Fig. 16 is a flowchart illustrating a method for manufacturing the expansion body assembly.
[Fig. 17] Fig. 17 is a front view illustrating a modification of the outer mold.
[Fig. 18] Fig. 18 is a side view illustrating a modification of the base material.
[Fig. 19] Fig. 19 is a side view illustrating a modification of the expansion body.
[Fig. 20] Fig. 20 is a side view illustrating a modification of the expansion body assembly.
[Fig. 21] Fig. 21 is a side view illustrating a modification of the medical device.

### Description of Embodiments

Hereinafter, an embodiment of the present invention will be described with reference to the drawings. Note that dimensional ratios in the drawings may be exaggerated and different from actual ratios for convenience of description. Herein, a side of a medical device 10 to be inserted into a biological lumen is referred to as "distal side" and a side to be operated is referred to as "proximal side".

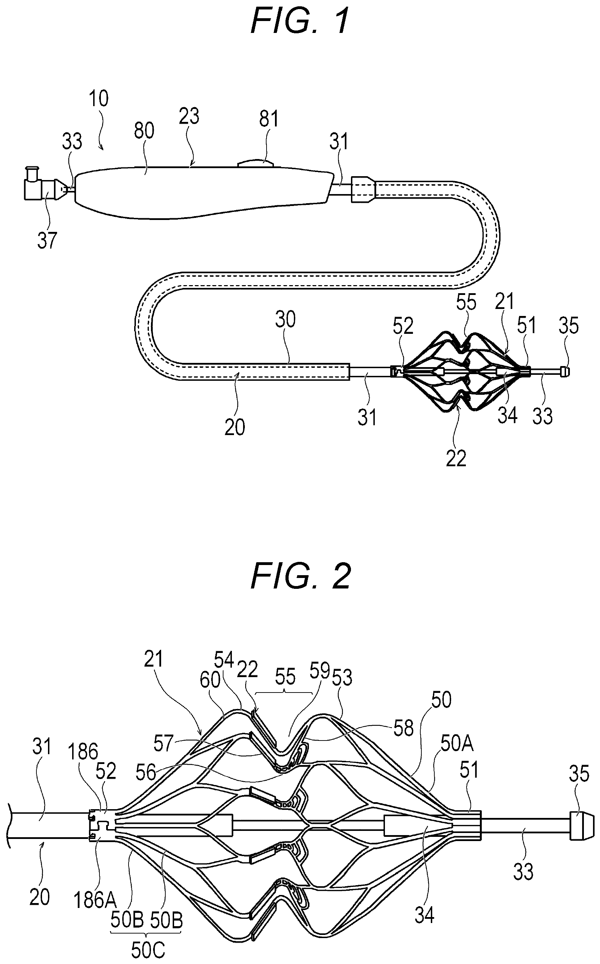

An expansion body 21 manufactured by the method for manufacturing the expansion body 21 according to the present embodiment is used in the medical device 10 illustrated in Fig. 1. The medical device 10 expands a puncture hole formed in an atrial septum of the heart of a patient to further perform a maintenance procedure to maintain the expanded puncture hole at the increased size. Note that the medical device 10 that can use the expansion body 21 is not particularly limited.

As illustrated in Figs. 1 and 2, the medical device 10 includes an elongated portion 20 extending from a proximal end to a distal end, the expansion body 21 disposed on a distal part of the elongated portion 20, an electrode unit 22 serving as an energy transfer element provided along the expansion body 21, and a hand operation unit 23 connected to a proximal part of the elongated portion 20.

The elongated portion 20 includes a shaft portion 31 holding the expansion body 21 at a distal part, a distal shaft portion 34 fixed to the distal part of the expansion body 21, an outer tube 30 that accommodates the shaft portion 31, a pulling shaft 33, and a distal chip 35 fixed to the distal end of the pulling shaft 33.

The shaft portion 31 is an elongated tubular body extending from the hand operation unit 23 to the proximal part of the expansion body 21. A proximal part of the shaft portion 31 is fixed to a distal part of the hand operation unit 23. A distal part of the shaft portion 31 is fixed to the proximal part of the expansion body 21. The distal shaft portion 34 is a tubular body fixed to the distal part of the expansion body 21. The distal shaft portion 34 is disposed on the distal side with respect to the shaft portion 31 with a space from the shaft portion 31.

The outer tube 30 is an elongated tubular body covering the shaft portion 31, and is movable forward and backward with respect to the shaft portion 31 in the axial direction (direction of the axial center of the elongated portion 20). The outer tube 30 can accommodate the expansion body 21 contracted in the radial direction therein in a state of being moved to the distal side of the elongated portion 20. The radial direction is a direction orthogonal to the axial center of the shaft portion 31. An operator moves the outer tube 30 that has accommodated the expansion body 21 to the proximal side, thereby being capable of exposing the expansion body 21 from the outer tube 30 and expanding the expansion body 21 in the radial direction by the restoring force of the expansion body 21.

The pulling shaft 33 is an elongated tubular body disposed inside the shaft portion 31 and the distal shaft portion 34, and is movable forward and backward with respect to the shaft portion 31 in the axial direction. The pulling shaft 33 protrudes from the distal end of the shaft portion 31 toward the distal side, and protrudes from the distal end of the expansion body 21 toward the distal side. A distal part of the pulling shaft 33 located distal of the expansion body 21 is fixed to the distal chip 35. A proximal part of the pulling shaft 33 is drawn out to the proximal side beyond the hand operation unit 23. A guide wire lumen is formed inside the pulling shaft 33 along the axial direction, and a guide wire can be inserted therethrough. The pulling shaft 33 can move relative to the shaft portion 31 along the axial direction.

The distal chip 35 is an annular member fixed to an outer peripheral surface of the distal part of the pulling shaft 33, and protrudes radially outward from the outer peripheral surface of the pulling shaft 33. The distal chip 35 is not fixed to the expansion body 21. The outer diameter of the distal chip 35 is larger than the inner diameter of the distal part of the expansion body 21. Therefore, the distal chip 35 can contact the distal part of the expansion body 21 from the distal side and pull the expansion body 21 in a proximal direction to apply a compressive force for compressing the expansion body 21 to the expansion body 21 along the axial direction of the shaft portion 31.

The expansion body 21 has a plurality of struts 50 extending in the axial direction and aligned in the circumferential direction. The struts 50 form a mesh-shaped structure by branching and joining along the axial direction. As a result, the expansion body 21 can expand and contract in a radial direction. The plurality of struts 50 includes a plurality of distal struts 50A located distal of a waist portion 55 having a reduced outer diameter and a plurality of proximal struts 50B located proximal of the waist portion 55. The plurality of proximal struts 50B forms two strut groups 50C. The plurality of proximal struts 50A included in each strut group 50C is connected to the same separation portion 186A described later.

The expansion body 21 is inclined to increase in the radial direction from both ends toward a central part in the axial direction. In addition, the expansion body 21 has a constricted shape with a sharply reduced outer diameter at the central part in the axial direction. That is, the expansion body 21 includes a ring-shaped distal connection portion 51 located on the distal side, a ring-shaped proximal connection portion 52 located on the proximal side, a distal-side projecting portion 53 located proximal of the distal connection portion 51 and protruding radially outward, a proximal-side projecting portion 54 located distal of the proximal connection portion 52 and protruding radially outward, and a waist portion 55 located between the distal-side projecting portion 53 and the proximal-side projecting portion 54 and having a reduced outer diameter.

The distal-side connection portion 51 is a ring-shaped part to which the distal ends of the plurality of struts 50 aligned in the circumferential direction are connected. The proximal-side connection portion 52 is a ring-shaped part to which the proximal ends of the plurality of struts 50 aligned in the circumferential direction are connected. The distal-side connection portion 51 is connected to the distal shaft portion 34. The proximal-side connection portion 52 is connected to the shaft portion 31.

The waist portion 55 includes a bottom 56 that is a part on the innermost side in the radial direction, a proximal-side upright portion 57 extending radially outward from a proximal end of the bottom 56, and a distal-side upright portion 58 extending radially outward from a distal end of the bottom 56. The waist portion 55 defines a reception space 59 that can receive a biological tissue when the expansion body 21 is expanded.

When the pulling shaft 33 slides in the proximal direction with respect to the shaft portion 31 to apply a compressive force to the expansion body 21, the distal-side upright portion 58 and the proximal-side upright portion 57 are brought close to each other, and both portions come in close contact with the biological tissue received in the reception space 59. The electrode unit 22 is disposed along the waist portion 55 at the proximal-side upright portion 57 so as to face the reception space 59. In other words, the electrode unit 22 is disposed along the expansion body 21 in an intermediate part of the expansion body 21 in the axial direction. In the present embodiment, ten electrode units 22 are disposed in the circumferential direction. Note that the electrode unit 22 may be disposed on the distal-side upright portion 58.

The expansion body 21 can be formed by, for example, cutting a single metal cylindrical member with laser. The expansion body 21 may be formed of a metal material. Examples of the metal material that may be used include a titanium-based (Ti-Ni, Ti-Pd, Ti-Nb-Sn, etc.) alloy, a copper-based alloy, stainless steel, β-titanium steel, and a Co-Cr alloy. Note that it is more preferable to use a shape memory alloy having high elasticity such as a nickel titanium alloy. However, the material of the expansion body 21 is not limited to the above materials, and other materials may be used.

The electrode units 22 are disposed on a conductive portion 60 of the expansion body 21 and are connected to an energy supply device (not illustrated), which is an external device, via the conductive portion 60. A highfrequency voltage is applied from the energy supply device to an electrode pair including two electrode units 22 via the conductive portion 60, and energy is applied between them. In other words, the electrode unit 22 is configured as a bipolar electrode.

The electrode units 22 are disposed on a surface of the proximal-side upright portion 57 facing the distal side at the time of expansion of the expansion body 21. The electrode units 22 are disposed on the proximal-side upright portion 57. Accordingly, when the waist portion 55 grips the atrial septum, the energy from the electrode units 22 is transferred to the atrial septum from the right atrium side. When the electrode units 22 are disposed on the distal-side upright portion 58, the energy from the electrode units 22 is transferred to the atrial septum from the left atrial side.

The hand operation unit 23 includes a housing 80 gripped by the operator and an operation portion 81 movable by the operator along the axial direction of the pulling shaft 33. The housing 80 is fixed to the proximal part of the shaft portion 31, and the operation portion 81 is fixed to the proximal part of the pulling shaft 33.

The medical device 10 is used in treatment to be performed on a patient suffering from chronic heart failure in which myocardial hypertrophy appears in the left ventricle of the heart H and stiffness (hardness) increases so that a blood pressure increases in the left atrium. The expansion body 21 of the medical device 10 is inserted halfway into a puncture hole in the atrial septum. Therefore, the edge of the puncture hole formed in the atrial septum is placed in the reception space 59 defined by the waist portion 55. When the pulling shaft 33 is pulled in the proximal direction with respect to the shaft portion 31, a compressive force is applied to the expansion body 21, so that the distal-side upright portion 58 and the proximal-side upright portion 57 are brought close to each other, and the electrode units 22 disposed on the proximal-side upright portion 57 are pressed against the biological tissue. In this state, the medical device 10 can apply high frequency energy to the edge of the puncture hole through the electrode units 22, thereby being capable of cauterizing (heating and cauterizing) the edge of the puncture hole with the high frequency energy. This results in making it possible to inhibit blockage of the puncture hole due to natural healing and maintain a size thereof.

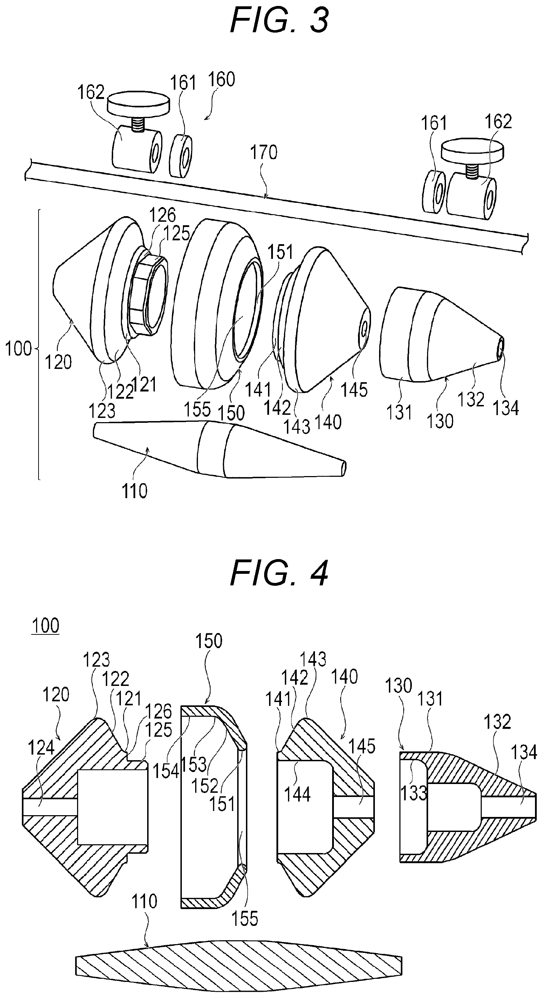

Next, a device for manufacturing the expansion body 21 will be described. As illustrated in Figs. 3 and 4, a mold 100, a core member 170, and a fixture 160 are used to manufacture the expansion body 21. The mold 100 includes a preform mold 110, a first inner mold 120 (inner mold), a second inner mold 130 (inner mold), a support inner mold 140 (inner mold), and an outer mold 150.

The core member 170 is a columnar member that can penetrate the first inner mold 120, the second inner mold 130, and the support inner mold 140 in order to support them. The fixture 160 includes a ring-shaped fixture 161 that can cover the end of the expansion body 21, and a positioning fixture 162 that can be fixed to the core member 170 with a screw or the like.

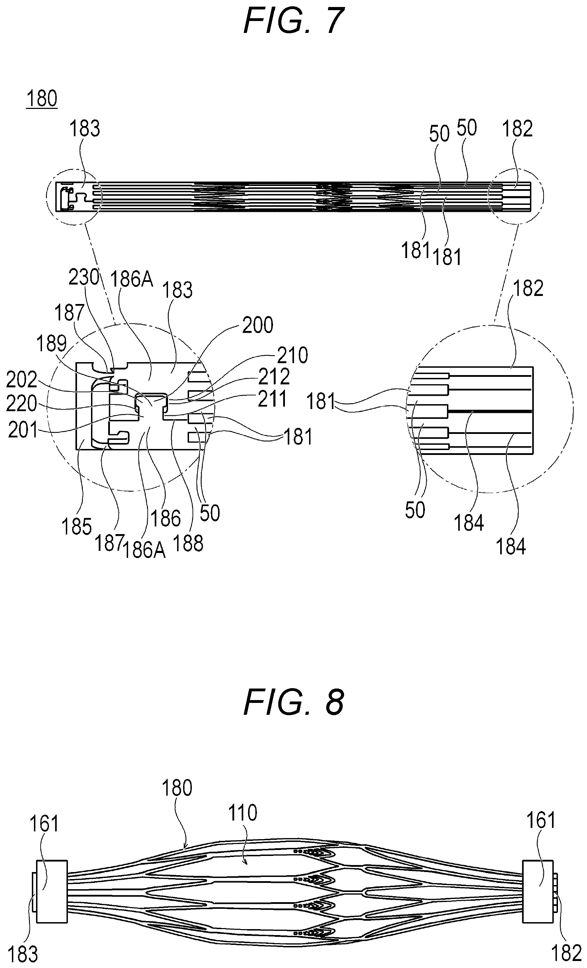

The first inner mold 120 is a mold that forms a half side positioned on one side (distal side or proximal side) in the axial direction from the bottom 56 of the expansion body 21, and can be inserted into a base material 180 (see Fig. 7) before being formed into the expansion body 21. The first inner mold 120 includes a first inner-mold recess 121 having a shape corresponding to the inner surface (radially inner surface) of the bottom 56 of the expansion body 21, a first inner-mold inclined portion 122 having a shape corresponding to the inner surface of the upright portion (the distal-side upright portion 58 and/or the proximal-side upright portion 57) of the expansion body 21, and a first inner-mold projecting portion 123 having a shape corresponding to the inner surface of the projecting portion (the distal-side projecting portion 53 and/or the proximal-side projecting portion 54) of the expansion body 21. The first inner mold 120 further includes a first connection portion 125 connectable to the second inner mold 130 and the support inner mold 140, a step portion 126 positioned between the first inner-mold recess 121 and the first connection portion 125, and a first through hole 124 which penetrates in the axial direction and into which the core member 170 can be inserted in close contact. The first connection portion 125 can be fitted and connected to a second connection portion 133 of the second inner mold 130 or a third connection portion 144 of the support inner mold 140 described later. The first inner-mold recess 121, the first inner-mold inclined portion 122, and the first inner-mold projecting portion 123 are formed to have a uniform outer diameter (circular cross section) in the circumferential direction around the central axis of the first through hole 124.

The second inner mold 130 is connected to the first inner mold 120 and can be inserted into the base material 180 before being formed into the expansion body 21 together with the first inner mold 120. The second inner mold 130 includes an outer diameter constant portion 131 having an outer diameter substantially the same as the inner diameter of the inner surface of the bottom 56 of the expansion body 21 (or the outer diameter of the first inner-mold recess 121 of the first inner mold 120) continuously in the axial direction from the side to be connected to the first inner mold 120, and a tapered portion 132 having an outer diameter tapered from the end of the outer diameter constant portion 131. The second inner mold 130 further includes a second connection portion 133 connectable to the first connection portion 125 of the first inner mold 120, and a second through hole 134 which penetrates in the axial direction and into which the core member 170 can be inserted in close contact. The first connection portion 125 can be fitted to the second connection portion 133. The outer diameter constant portion 131 and the tapered portion 132 are formed to have a uniform outer diameter (circular cross section) in the circumferential direction around the central axis of the second through hole 134.

The support inner mold 140 is connected to the first inner mold 120 and can be inserted into the base material 180 before being formed into the expansion body 21 together with the first inner mold 120. The support inner mold 140 includes a support inner-mold recess 141 having a shape corresponding to the bottom 56 of the expansion body 21, a support inner-mold inclined portion 142 having a shape corresponding to the upright portion (the distal-side upright portion 58 or the proximal-side upright portion 57) of the expansion body 21, and a support inner-mold projecting portion 143 having a shape corresponding to the projecting portion (the distal-side projecting portion 53 or the proximal-side projecting portion 54) of the expansion body 21. The support inner mold 140 further includes a third connection portion 144 connectable to the first connection portion 125 of the first inner mold 120, and a third through hole 145 which penetrates in the axial direction and into which the core member 170 can be inserted in close contact. The first connection portion 125 can be fitted to the third connection portion 144. The third connection portion 144 has the same shape as the second connection portion 133 that can be similarly connected to the first connection portion 125. The support inner-mold recess 141, the support inner-mold inclined portion 142, and the support inner-mold projecting portion 143 are formed to have a uniform outer diameter (circular cross section) in the circumferential direction around the central axis of the third through hole 145.

The outer mold 150 is a substantially ring-shaped mold that can be placed so as to surround the first inner mold 120 on the radially outer side of the first inner mold 120, and is used to hold the base material 180 with the first inner mold 120 and to shape the base material 180. The outer mold 150 includes an outer-mold projecting portion 151 having a shape corresponding to the outer surface (radially outer surface) of the bottom 56 of the expansion body 21, an outer-mold inclined portion 152 having a shape corresponding to the outer surface of the upright portion (the distal-side upright portion 58 and/or the proximal-side upright portion 57) of the expansion body 21, an outer-mold recess 153 having a shape corresponding to the outer surface of the projecting portion (the distal-side projecting portion 53 and/or the proximal-side projecting portion 54) of the expansion body 21, and an inner diameter constant portion 154 having an inner diameter substantially equal to the outer-mold recess 153 continuously in the axial direction. The outer mold 150 further includes a fourth through hole 155 into which the first inner mold 120 can be inserted. The outer-mold projecting portion 151 protrudes radially inward and has the smallest inner diameter of the outer mold 150. The outer mold 150 has a uniform inner diameter (circular cross section) in the circumferential direction around the central axis of the fourth through hole 155.

The preform mold 110 is a spindle-shaped mold having a maximum outer diameter smaller than the outer diameter of the first inner-mold recess 121.

The mold 100 can be formed of a metal material. As the metal material, for example, stainless steel, carbon steel, copper, brass, or the like can be used.

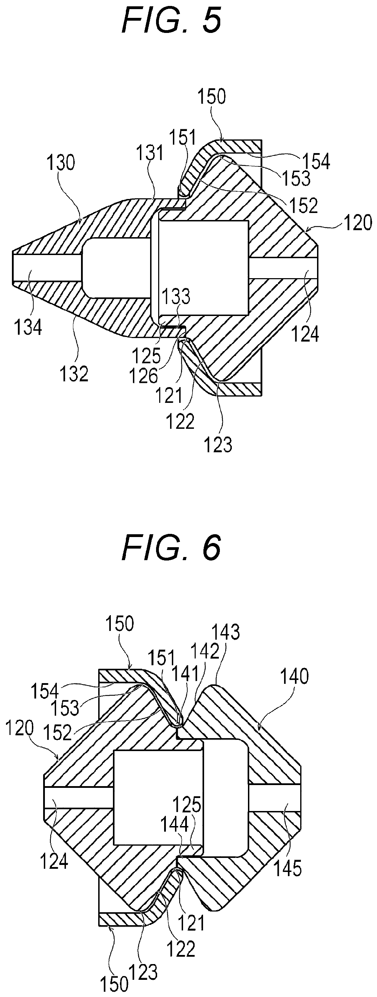

The first inner mold 120, the second inner mold 130, and the outer mold 150 can be connected as in a first use example illustrated in Fig. 5. Further, the first inner mold 120, the support inner mold 140, and the outer mold 150 can be connected as in a second use example illustrated in Fig. 6.

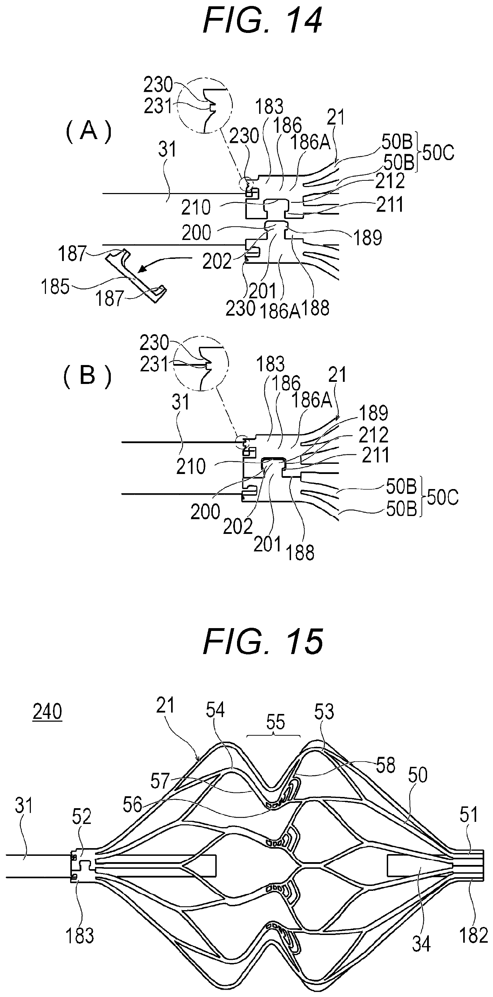

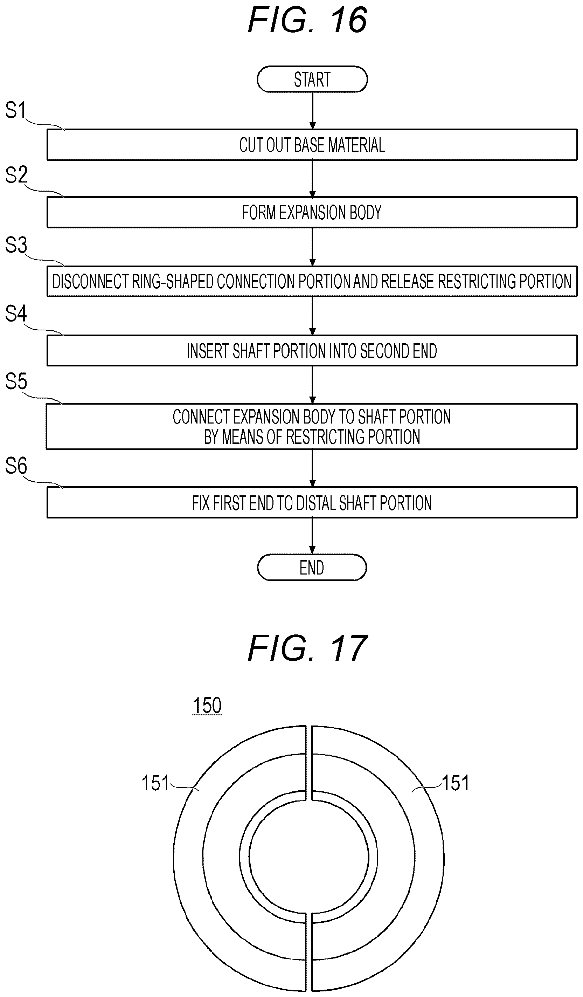

Next, a method for manufacturing the expansion body 21 and an expansion body assembly 240 according to the present embodiment will be described with reference to a flowchart illustrated in Fig. 16. As illustrated in Fig. 15, the expansion body assembly 240 is a member in which the shaft portion 31 and the distal shaft portion 34 are connected to the expansion body 21, and does not include the electrode unit 22, the pulling shaft 33, the hand operation unit 23, and the like. In the expansion body assembly 240, the distal shaft portion 34 may not be connected.

First, a manufacturer prepares a metal cylindrical member before being formed into the expansion body 21, and cuts out and creates the base material 180 by laser processing or the like as illustrated in Fig. 7 (step S1). In the base material 180, a plurality of cut portions 181 penetrating from the outer peripheral surface to the inner peripheral surface are formed, and struts 50 are formed between the adjacent cut portions 181. In the base material 180, a first end 182 and a second end 183 are formed.

The first end 182 is a section to be the distal-side connection portion 51 of the expansion body 21, and has a plurality of first slits 184 extending from the distal end face of the base material 180 to the cut portions 181 positioned between the struts 50 adjacent in the circumferential direction.

The second end 183 includes a section to be the proximal-side connection portion 52 of the expansion body 21, and includes a ring-shaped connection portion 185 located at the proximal end of the base material 180, a fixing portion 186 located distal of (or on the first end 182 side) of the ring-shaped connection portion 185, and a cuttable portion 187 connecting each fixing portion 186 and the ring-shaped connection portion 185. The ring-shaped connection portion 185 is a ring-shaped section formed 360 degrees.

The fixing portion 186 has at least one (two in the present embodiment) second slit 188 and two separation portions 186A separated by the two second slits 188 in the circumferential direction. The two separation portions 186A are connected by means of a restricting portion 189 that can connect the separation portions 186A by engagement and can separate them from each other. The restricting portion 189 is formed in the second slits 188. Note that the number of second slits 188 may be one. In this case, the fixing portion 186 does not have the separation portion 186A, and can be opened to widen one first slit 188.

In the restricting portion 189, for example, a T-shaped protrusion 200 can be engaged with and connected to a recess 210 that is recessed in a T shape. The protrusion 200 has a base 201 formed at one end in the circumferential direction and a top 202 formed at the other end in the circumferential direction and longer than the base 201 in the axial direction of the expansion body 21. The recess 210 has an opening top 211 that receives the base 201 of the protrusion 200 and an opening bottom 212 that communicates with the opening top 211 and receives the top 202 of the protrusion 200. The length of the top 202 in the circumferential direction is shorter than the length of the opening bottom 212 in the circumferential direction. The proximal part of the expansion body 21 is fixed to the shaft portion 31 in a state where the protrusion 200 is fitted in the recess 210 and a gap 220 in the circumferential direction is formed between the top 202 of the protrusion 200 and the opening bottom 212 of the recess 210 (see Fig. 14(B)). In the present embodiment, the top 202 protrudes to both the distal side and the proximal side with respect to the base 201, but may protrude to only one of the distal side and the proximal side with respect to the base 201.

The proximal end of the fixing portion 186 has a depressed portion 230 that is recessed toward the distal end of the expansion body 21. The cuttable portion 187 is connected to the fixing portion 186 in the recessed part of the depressed portion 230. The cuttable portion 187 is formed to be thin from the ring-shaped connection portion 185 toward the depressed portion 230. Therefore, the cuttable portion 187 can be cut at a position closer to the depressed portion 230 than the ring-shaped connection portion 185. The cuttable portion 187 forms a cut mark 231 in the recessed part of the depressed portion 230 when being cut.

The ring-shaped connection portion 185 prevents the release of the connection state between the protrusion 200 and the recess 210 of the restricting portion 189 to inhibit the second slit 188 of the fixing portion 186 from being opened (the plurality of separation portions 186A from being separated), and appropriately maintains the shape of the base material 180 until the expansion body 21 is manufactured. Note that the restricting portion 189 is not particularly limited as long as it can connect the opposing edges of the second slit 188 of the fixing portion 186 to each other and can separate them from each other.

Next, the manufacturer forms the expansion body 21 from the member 180 (step S2). For this purpose, the manufacturer forcibly widens the first slit 184 in the first end 182 of the base material 180, and inserts the preform mold 110 into the base material 180 from the widened opening in the first end 182 as illustrated in Fig. 8. Next, the manufacturer covers the outside of the first end 182 and the second end 183 with the ring-shaped fixtures 161 to keep the first slit 184 in the first end 182 and the second slit 188 in the second end 183 closed, and applies heat treatment (heating and heat dissipation). Thus, the base material 180 is given the shape of the preform mold 110.

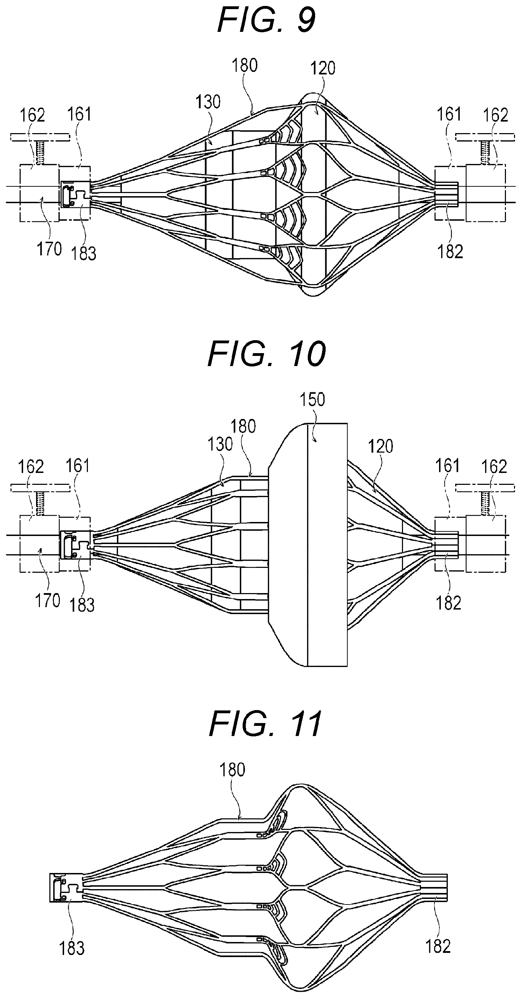

Next, the manufacturer removes the ring-shaped fixtures 161 from the first end 182 and the second end 183, forcibly widens the slit in the first end 182 of the base material 180, and takes out the preform mold 110 from the widened opening of the first end 182. Subsequently, as illustrated in Figs. 5 and 9, the manufacturer inserts the second inner mold 130 and the first inner mold 120 into the base material 180 from the opening of the first end 182. At this time, the first connection portion 125 and the second connection portion 133 are connected, the core member 170 is inserted into the first through hole 124 and the second through hole 134, the second inner mold 130 is placed on a half side on the second end 183 side of the base material 180, and the first inner mold 120 is placed on a half side on the first end 182 side of the base material 180. Next, as illustrated in Fig. 10, the manufacturer puts and fixes the ring-shaped fixture 161 on the first end 182, restricts the movement of the first end 182 in the axial direction by the positioning fixture 162, and puts the outer mold 150 on the outside of the first inner mold 120 from the proximal side. As a result, a half side close to the first end 182 of the base material 180 is pushed outward by the first inner mold 120, and deformation to the outside is limited by the outer mold 150. Next, the manufacturer covers the second end 183 with the ring-shaped fixture 161 to close the second slit 188, and restricts the movement of the second end 183 in the axial direction by the positioning fixture 162. Next, the manufacturer applies a heat treatment. As a result, the base material 180 is given the shapes of the first inner mold 120, the second inner mold 130, and the outer mold 150. In particular, the base material 180 is given the shapes (see Fig. 2) of the distal-side upright portion 58 and the distal-side projecting portion 53 from the bottom 56 of the expansion body 21 by the first inner mold 120 and the outer mold 150. Further, the base material 180 is given, to some extent, the shape from the bottom 56 to the proximal side of the expansion body 21 by the second inner mold 130.

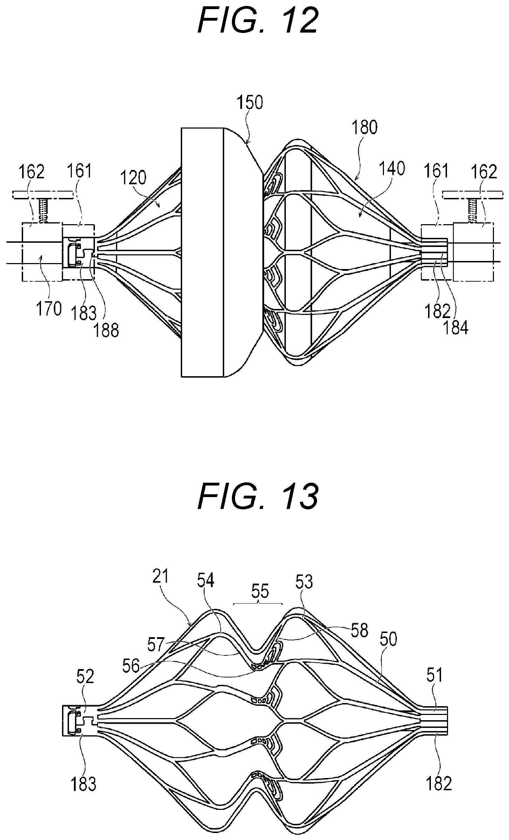

Next, as illustrated in Fig. 11, the manufacturer temporarily removes the outer mold 150, the first inner mold 120, the second inner mold 130, the core member 170, and the fixture 160 from the base material 180. Subsequently, the manufacturer again widens the first slit 184 in the first end 182 of the base material 180, inserts the first inner mold 120 into the base material 180 from the opening of the first end 182 as illustrated in Figs. 6 and 12, and places the first inner mold 120 on a half side on the second end 183 side. Next, the manufacturer inserts the core member 170 into the first through hole 124, puts the ring-shaped fixture 161 on the second end 183 to close the second slit 188, restricts the movement of the second end 183 in the axial direction by the positioning fixture 162, and covers the outside of the base material 180 that covers the first inner mold 120 with the outer mold 150 from the distal side. As a result, a half side close to the second end 183 of the base material 180 is pushed outward by the first inner mold 120, and deformation to the outside is limited by the outer mold 150.

Subsequently, the manufacturer again widens the first slit 184 in the first end 182 of the base material 180, inserts the support inner mold 140 into the base material 180 from the opening of the first end 182, and places the support inner mold 140 on a half side on the first end 182 side that has already been shaped. At this time, the manufacturer connects the third connection portion 144 of the support inner mold 140 to the first connection portion 125 of the first inner mold 120, and inserts the core member 170 through the third through hole 145. Next, the manufacturer puts the ring-shaped fixture 161 on the first end 182 to close the first slit 184, and restricts the movement of the first end 182 in the axial direction by the positioning fixture 162. Next, the manufacturer applies a heat treatment. As a result, the base material 180 is given the shapes of the first inner mold 120, the support inner mold 140, and the outer mold 150. In particular, the base material 180 is given the shapes (see Fig. 2) of the proximal-side upright portion 57 and the proximal-side projecting portion 54 from the bottom 56 of the expansion body 21 by the first inner mold 120 and the outer mold 150.

Next, as illustrated in Fig. 13, the manufacturer removes the outer mold 150, the first inner mold 120, the support inner mold 140, the core member 170, and the fixture 160 from the base material 180. Thus, the expansion body 21 is created.

Before connecting the expansion body 21 to the shaft portion 31, the manufacturer cuts the cuttable portion 187 to disconnect the ring-shaped connection portion 185 from the fixing portion 186 as illustrated in Fig. 14(A). Next, the manufacturer removes the protrusion 200 of the restricting portion 189 at the second end 183 from the recess 210 to release the restricting portion 189, and separates the two separation portions 186A so as to widen the second slit 188 (step S3). With this process, the shaft portion 31 can be easily covered with the second end 183. Next, the manufacturer inserts the shaft portion 31 into the second end 183 (step S4). Next, as illustrated in Fig. 14(B), the manufacturer engages the protrusion 200 of the restricting portion 189 with the recess 210 to fix the expansion body 21 to the shaft portion 21 (step S5). At this time, the circumferential position of the top 202 of the protrusion 200 in the opening bottom 212 of the recess 210 can be adjusted within the area of the gap 220. Therefore, even if the outer diameter of the shaft portion 31 varies, the second end 183 can be connected to the shaft portion 31. Thus, the second end 183 is firmly connected to the shaft portion 31. The first end 182 divided into a plurality of sections can be gathered and easily connected to the outer peripheral surface of the shaft portion 31.

Next, as illustrated in Fig. 15, the manufacturer gathers and fixes the first end 182 (distal strut) divided into a plurality of sections on the outer peripheral surface of the distal shaft portion 34 in order to prevent the distal part of the expansion body 21 from spreading by the first slit 184 (step S6). As a result, the expansion body assembly 240 is manufactured. The first end 182 may be fixed to the distal shaft portion 34 before the second end 183 is fixed to the shaft portion 31 or the ring-shaped connection portion 185 is disconnected from the second end 183.

As described above, the method for manufacturing the expansion body 21 according to the present embodiment is a method for manufacturing an expansion body 21 that includes a plurality of struts 50 extending along an axial direction and aligned in a circumferential direction around the axial direction, and has, in the middle in the axial direction, a waist portion 55 having a reduced outer diameter, the method including expanding an inside of a base material 180 that has a tubular shape and includes the plurality of struts 50 using an inner mold, and restricting an outside of the base material 180 with an outer mold 150 in order to form a bottom 56 having the smallest outer diameter of the waist portion 55 and an upright portion (the distal-side upright portion 58 and/or the proximal-side upright portion 57) extending radially outward from the bottom 56. With this configuration, in the method for manufacturing the expansion body 21, the bottom 56 and the upright portion of the waist portion 55 are formed using the inner mold and the outer mold 150, and thus, the method can reduce the strain generated in the expansion body 21 due to the rapid deformation during manufacture and inhibit the breakage of the expansion body 21.

The method for manufacturing the expansion body 21 includes a first step of forming a half side positioned on one side in the axial direction from the bottom 56 of the expansion body 21 together with the bottom 56 using a first inner mold 120 and the outer mold 150, and a second step of forming another half side positioned on a side opposite to the one side of the expansion body 21. With this configuration, in the method for manufacturing the expansion body 21, the two sides of the expansion body 21 are formed in separate steps, and thus, the method can reduce the strain generated in the expansion body 21 and inhibit the breakage of the expansion body 21.

The inner mold includes a first inner mold 120, and in the method for manufacturing the expansion body 21, the outside of the base material 180 into which the first inner mold 120 has been inserted is covered with the outer mold 150 that has a ring shape and that is not a split mold in the first step and the second step. When the outer mold 150 is a split mold, each piece of the split mold may deform differently due to repeated use. On the other hand, the outer mold 150 has a ring shape, and thus, deformation of the outer mold 150 due to repeated use can be reduced, whereby the expansion body 21 having an appropriate shape can be manufactured.

In the method for manufacturing the expansion body 21, the same molds (first inner mold 120 and outer mold 150) are used in the first step and the second step to form both of the half sides located on both sides from the bottom 56 in the axial direction. With this configuration, the two half sides located across the bottom 56 can be formed in the same shape.

In the method for manufacturing the expansion body 21, an inside of the half side formed in the first step is supported with a support inner mold 140 including a projecting portion projecting radially outward in the second step. With this configuration, in the second step, deformation of the shape of the half side formed in the first step can be reduced, so that the shape of the half side formed in the first step can be appropriately maintained.

In the method for manufacturing the expansion body 21, the vicinity of the bottom 56 inside the other half side to be formed in the second step is expanded in the first step using a second inner mold 130 including an outer diameter constant portion 131 that continuously has an outer diameter substantially equal to the outer diameter of the bottom 56 in the axial direction. With this configuration, when the half side on one side is formed in the first step, the other half side can be deformed to the same extent as the bottom 56. As a result, it is possible to reduce the strain generated in the expansion body 21 in the second step and to inhibit the breakage of the expansion body 21.

In the method for manufacturing the expansion body 21, the vicinity of the bottom 56 inside the other half side to be formed in the second step is expanded in the first step using a second inner mold 130 including an outer diameter constant portion 131 that continuously has an outer diameter substantially equal to the outer diameter of the bottom 56 in the axial direction, and in the second step, the other half side is formed using the first inner mold 120 and the outer mold 150 and an inside of the half side formed in the first step is supported with a support inner mold 140 including a projecting portion projecting radially outward. With this configuration, the first inner mold 120 and the outer mold 150 can be commonly used in the first step and the second step, whereby the two sides located across the bottom 56 can be easily formed in the same shape.

The method for manufacturing the expansion body 21 includes expanding the base material 180 into a spindle shape using a preform mold 110 having a maximum outer diameter smaller than an inner diameter of the bottom 56 of the first inner mold 120 before the first step. With this configuration, the base material 180 can be deformed to some extent before the first step. As a result, it is possible to reduce the strain generated in the expansion body 21 in the first step and to inhibit the breakage of the expansion body 21.

The method for manufacturing the expansion body 21 includes a step of forming a plurality of first slits 184 along the axial direction in a first end 182 in the axial direction of the base material 180 before the first step, and in the first step and the second step, the first end 182 is widened and the inner mold is inserted into the base material from the first end 182. With this configuration, the first end 182 is easily widened, whereby it is easy to insert the inner mold from the first end 182 into the base material 180 and to place the inner mold at a desired position.

The method for manufacturing the expansion body 21 includes disconnecting, from the base material 180, a ring-shaped connection portion 185 that has been placed at a second end 183 on a side opposite to the first end 182 of the base material 189 for maintaining a tubular shape of the second end 183, after the second step. With this configuration, the method for manufacturing the expansion body 21 can easily and firmly fix the second end 183 to the distal part of the shaft portion 31.

The method for manufacturing the expansion body 21 includes a step of forming a second slit 188 in the second end 183 along the axial direction and forming a restricting portion 189 for restricting the widening of the second slit 188. With this configuration, the method for manufacturing the expansion body 21 facilitates the insertion of the shaft portion 31 to be connected into the second end 183 by releasing the restricting portion 189. Further, after the shaft portion 31 is inserted into the second end 183, the restricting portion 189 restricts the widening of the second slit 188, whereby the second end 183 can be easily fixed to the shaft portion 31.

Further, the method for manufacturing the expansion body assembly 240 according to the present embodiment includes: cutting out a base material 180 having a tubular shape to form a plurality of struts 50 extending in the axial direction of the base material 180 and aligned in the circumferential direction around the axial direction and to form, at a second end 183 on a side opposite to a first end in the axial direction of the base material 180, a fixing portion 186 having a tubular shape and including a second slit 188 along the axial direction and a restricting portion 189 for restricting widening of the second slit 188, and a ring-shaped connection portion 185 connected to the fixing portion 186 and maintaining the tubular shape of the fixing portion 186; deforming the plurality of struts 50 of the base material 180 in the radial direction using a mold 100 to form an expansion body 21 with a waist portion 55 having a reduced outer diameter in the middle in the axial direction; disconnecting the ring-shaped connection portion 185 from the fixing portion 186 and widening the second slit 188 by removing restriction by the restricting portion 189 after forming the expansion body 21; inserting a shaft portion 31 that is long into the expansion body 21; and after covering a distal part of the shaft portion 31 with the fixing portion 186 at the second end 183, restricting the widening of the second slit 188 by the restricting portion 189 and fixing the fixing portion 186 at the second end 183 to the distal part of the shaft portion 31. With this configuration, in the method for manufacturing the expansion body assembly 240, the tubular shape of the fixing portion 186 at the second end 183 which is more likely to be widened by the second slit 188 can be suppressed by the ring-shaped connection portion 185, until the expansion body 21 is created from the base material 180 and the expansion body 21 is fixed to the shaft portion 31. Therefore, the expansion body 21 and the expansion body assembly 240 can be easily manufactured. In addition, the ring-shaped connection portion 185 is disconnected after the expansion body 21 is formed, and thus, the second slit 188 can be widened. Accordingly, it is easy to insert the shaft portion 31 into the fixing portion 186 at the second end 183 and to fix the fixing portion 186 at the second end 183 to the shaft portion 31 by means of the restricting portion 189.

In addition, the medical device 10 according to the present embodiment includes an expansion body 21 including a plurality of struts 50 extending along an axial direction and aligned in a circumferential direction around the axial direction, and a shaft portion 31 that is long and that is connected to a proximal part of the expansion body 21, wherein the proximal part of the expansion body 21 includes a fixing portion 186 having a tubular shape, provided with at least one proximal slit (second slit 188) along the axial direction, and capable of opening and closing the proximal slit, one of two edges of the fixing portion 186 located across the proximal slit is formed with a protrusion 200 protruding to another edge in the circumferential direction, while the other edge is provided with a recess 210 recessed to be engaged with the protrusion 200, the proximal slit being formed to include a boundary between the protrusion 200 and the recess 210, the protrusion 200 includes a base 201 formed at one end in the circumferential direction, and a top 202 formed on another end in the circumferential direction and longer than the base 201 in the axial direction of the expansion body 21, the recess 210 includes an opening top 211 that receives the base 201 of the protrusion 200 and an opening bottom 212 that communicates with the opening top 211 and receives the top 202 of the protrusion 200, the top 202 has a length in the circumferential direction shorter than the length in the circumferential direction of the opening bottom 212, and the proximal part of the expansion body 21 is fixed to the shaft portion 31 in a state in which the protrusion 200 is fitted into the recess 210 and a gap 220 in the circumferential direction is formed between the top 202 of the protrusion 200 and the opening bottom 212 of the recess 210. With this configuration, in the medical device 10, the top 202 of the protrusion 200 of the expansion body 21 is engaged with the opening bottom 212 of the recess 210 to fix the expansion body 21 to the shaft portion 31. In addition, since the gap 220 in the circumferential direction is formed between the top 202 of the protrusion 200 and the opening bottom 212 of the recess 210, the second end 183 can be satisfactorily fixed to the shaft portion 31 even if the outer diameter of the shaft portion 31 varies.

The fixing portion 186 has, at a proximal end, a cut mark 231 obtained by cutting the ring-shaped connection portion 185. With this configuration, the medical device 10 can maintain the tubular shape of the fixing portion 186 by the ring-shaped connection portion 185 until necessary at the time of manufacturing, and thus is easy to manufacture.

The proximal end of the fixing portion 186 includes a depressed portion 230 that is recessed toward a distal end of the expansion body 21 and is provided with the cut mark 231 inside. With this configuration, it is possible to prevent the medical device 10 from damaging another medical device or biological tissue due to the cut mark 231 contacting therewith.

The plurality of struts 50 includes a plurality of sets of strut groups 50C extending toward the proximal part of the expansion body 21, the fixing portion 186 includes at least one separation portion 186A separated in the circumferential direction by the proximal slit (second slit 188), and the at least one separation portion 186A is each connected to a proximal end of a corresponding one of the strut groups 50C. With this configuration, in the medical device 10, the separation portion 186A is formed in accordance with the strut groups 50C, whereby it is possible to prevent the occurrence of distortion of the shape of the expansion body 21 due to the excessive number of separation portions 186A.

Distal parts of the plurality of struts 50 include a plurality of distal struts 50A that extends in the axial direction and is independent from each other in the circumferential direction, and the distal part of the expansion body 21 includes a distal shaft portion 34 that fixes the plurality of distal struts 50A in such a manner that two adjacent distal struts 50A are not separated from each other in the circumferential direction. With this configuration, in the medical device 10, the plurality of independent distal struts 50A is fixed to the distal shaft portion 34, whereby it is possible to prevent the occurrence of distortion of the shape of the distal part of the expansion body 21.

Note that the present invention is not limited to the embodiment described above, and those skilled in the art can make various modifications within the technical idea of the present invention. For example, the outer mold 150 may have split molds 151 that can be divided in the circumferential direction as illustrated in Fig. 17. As a result, the split molds 151 of the outer mold 150 can be separately placed from the radially outer side with respect to the first inner mold 120, so that the outer mold 150 can be easily placed.

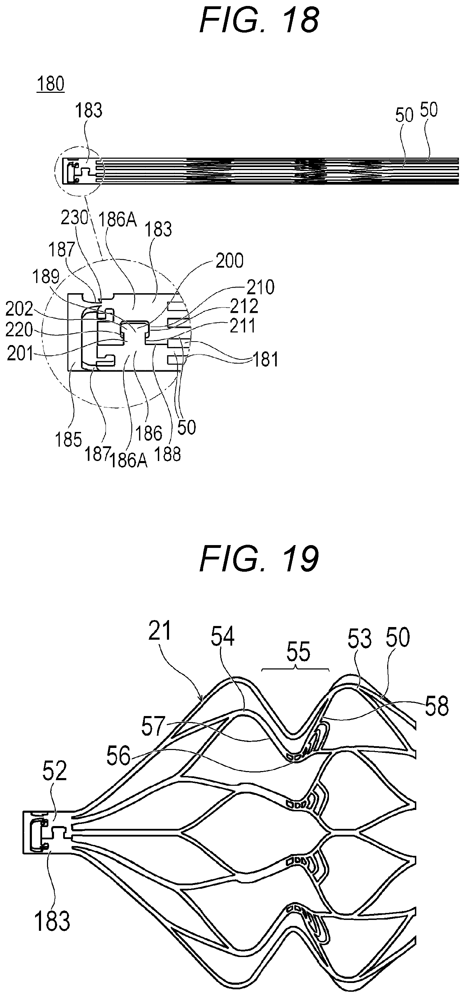

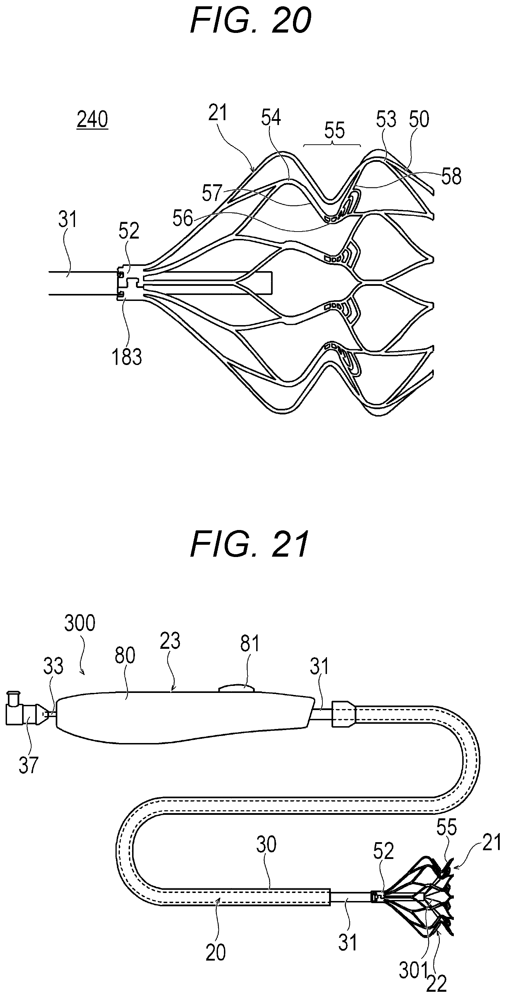

In addition, as illustrated in Fig. 18, the distal part of the base material 180 (expansion body 21) may not have the first end where the struts 50 are gathered. In this case, in the distal part of the expansion body 21 to be created, the distal ends of the plurality of struts 50 spread as illustrated in Fig. 19. When the shaft portion 31 is inserted and fixed to a second end 52 of the expansion body 21, the expansion body assembly 240 is created as illustrated in Fig. 20. The expansion body assembly 240 does not include a distal shaft portion. Fig. 21 illustrates a medical device 300 manufactured using the expansion body assembly 240. The medical device 300 includes at least one pulling wire 301 connected to the operation portion 81 instead of the pulling shaft. The distal end of the pulling wire 301 is fixed to the waist portion 55. Therefore, the diameter of the waist portion 55 can be adjusted by moving the pulling wire 301 in the proximal direction with the operation portion 81.

Further, in the method for manufacturing the expansion body assembly 240, the method for creating the expansion body 21 is not limited to the method illustrated in Fig. 13. For example, the expansion body 21 may be created by inserting an inner mold corresponding to the shape of the expansion body 21 inside the base material 180 and narrowing a position corresponding to the waist portion 55 of the base material 180 with a wire or the like.

Note that the present application is based on Japanese Patent Application No. 2023-169566 filed on September 29, 2023, the entire disclosed content of which is incorporated herein by reference.

### Reference Signs List

- 10, 300: Medical device
- 21: Expansion body
- 31: Shaft portion
- 34: Distal shaft portion
- 50: Strut
- 50A: Distal strut
- 50B: Proximal strut
- 50C: Strut group
- 55: Waist portion
- 56: Bottom
- 57: Proximal-side upright portion (upright portion)
- 58: Distal-side upright portion (upright portion)
- 100: Mold
- 110: Preform mold
- 120: First inner mold (inner mold)
- 130: Second inner mold (inner mold)
- 131: Outer diameter constant portion
- 140: Support inner mold (inner mold)
- 150: Outer mold
- 180: Base material
- 181: Cut portion
- 182: First end
- 183: Second end
- 184: First slit
- 185: Ring-shaped connection portion
- 186: Fixing portion
- 186A: Separation portion
- 187: Cuttable portion
- 188: Second slit (proximal slit)
- 189: Restricting portion
- 200: Protrusion
- 201: Base
- 202: Top
- 210: Recess
- 211: Opening top
- 212: Opening bottom
- 220: Gap
- 230: Depressed portion
- 231: Cut mark
- 240: Expansion body assembly

## Claims

1. A method for manufacturing an expansion body that includes a plurality of struts extending along an axial direction and aligned in a circumferential direction around the axial direction, and has, in a middle in the axial direction, a waist portion having a reduced outer diameter, the method comprising
expanding an inside of a base material that has a tubular shape and is formed with a plurality of cuts using an inner mold, and restricting an outside of the base material with an outer mold in order to form a bottom having a smallest outer diameter of the waist portion and an upright portion extending radially outward from the bottom.

2. The method for manufacturing an expansion body according to claim 1, wherein
the inner mold includes a first inner mold, the method including
a first step of forming a half side positioned on one side in the axial direction from the bottom of the expansion body together with the bottom using the first inner mold and the outer mold, and
a second step of forming another half side positioned on a side opposite to the one side of the expansion body.

3. The method for manufacturing an expansion body according to claim 2, wherein the outside of the base material into which the first inner mold has been inserted is covered with the outer mold that is not a split mold and that has a ring shape in the first step and the second step.

4. The method for manufacturing an expansion body according to claim 2, wherein the first inner mold and the outer mold are commonly used in the first step and the second step to form both of the half sides located on both sides from the bottom in the axial direction.

5. The method for manufacturing an expansion body according to claim 2, wherein an inside of the half side formed in the first step is supported with a support inner mold including a projecting portion projecting radially outward in the second step.

6. The method for manufacturing an expansion body according to claim 2, wherein, in the first step, a vicinity of the bottom inside the other half side to be formed in the second step is expanded using a second inner mold including an outer diameter constant portion that continuously has an outer diameter substantially equal to an outer diameter of the bottom in the axial direction.

7. The method for manufacturing an expansion body according to claim 2, wherein
a vicinity of the bottom inside the other half side to be formed in the second step is expanded in the first step using a second inner mold including an outer diameter constant portion that continuously has an outer diameter substantially equal to an outer diameter of the bottom in the axial direction, and
in the second step, the other half side is formed using the first inner mold and the outer mold and an inside of the half side formed in the first step is supported with a support inner mold including a projecting portion projecting radially outward.

8. The method for manufacturing an expansion body according to claim 2, further comprising a step of expanding the base material into a spindle shape using a preform mold having a maximum outer diameter smaller than an inner diameter of the bottom of the first inner mold before the first step.

9. The method for manufacturing an expansion body according to claim 2, further comprising a step of forming a plurality of first slits along the axial direction in a first end in the axial direction of the base material before the first step, wherein
in the first step and the second step, the first end is widened and the inner mold is inserted into the base material from the first end.

10. The method for manufacturing an expansion body according to claim 9, further comprising disconnecting, from the base material, a ring-shaped connection portion that has been placed at a second end on a side opposite to the first end of the base material for maintaining a tubular shape of the second end, after the second step.

11. The method for manufacturing an expansion body according to claim 9 or 10, further comprising a step of forming a second slit in the second end along the axial direction and forming a restricting portion for restricting widening of the second slit.

12. A method for manufacturing an expansion body assembly comprising:
cutting out a base material having a tubular shape to form a plurality of struts extending in an axial direction of the base material and aligned in a circumferential direction around the axial direction and to form, at a second end on a side opposite to a first end in the axial direction of the base material, a fixing portion having a tubular shape and including a second slit along the axial direction and a restricting portion for restricting widening of the second slit, and a ring-shaped connection portion connected to the fixing portion and maintaining the tubular shape of the fixing portion;
deforming the plurality of struts of the base material in a radial direction using a mold to form an expansion body with a waist portion having a reduced outer diameter in a middle in the axial direction;
disconnecting the ring-shaped connection portion from the fixing portion and widening the second slit by removing restriction by the restricting portion after forming the expansion body;
inserting a shaft portion that is long into the expansion body; and
after covering a distal part of the shaft portion with the second end, restricting widening of the second slit by the restricting portion and fixing the second end to the distal part of the shaft portion.

13. A medical device comprising:
an expansion body including a plurality of struts extending along an axial direction and aligned in a circumferential direction around the axial direction; and
a shaft portion that is long and that is connected to a proximal part of the expansion body, wherein
the proximal part of the expansion body includes a fixing portion having a tubular shape, provided with at least one proximal slit along the axial direction, and capable of opening and closing the proximal slit,
one of two edges of the fixing portion located across the proximal slit is formed with a protrusion protruding to another edge in the circumferential direction, while the other edge is provided with a recess recessed to be engaged with the protrusion, the proximal slit being formed to include a boundary between the protrusion and the recess,
the protrusion includes a base formed at one end in the circumferential direction, and a top formed on another end in the circumferential direction and longer than the base in the axial direction of the expansion body,
the recess includes an opening top that receives the base of the protrusion and an opening bottom that communicates with the opening top and receives the top of the protrusion,
the top has a length in the circumferential direction shorter than a length in the circumferential direction of the opening bottom, and
the proximal part of the expansion body is fixed to the shaft portion in a state in which the protrusion is fitted into the recess and a gap in the circumferential direction is formed between the top of the protrusion and the opening bottom of the recess.

14. The medical device according to claim 13, wherein the fixing portion has, at a proximal end, a cut mark obtained by cutting the ring-shaped connection portion.

15. The medical device according to claim 14, wherein the proximal end of the fixing portion includes a depressed portion that is recessed toward a distal end of the expansion body and is provided with the cut mark inside.

16. The medical device according to claim 13, wherein
the plurality of struts includes a plurality of sets of strut groups extending toward the proximal part of the expansion body,
the fixing portion includes at least one separation portion separated in the circumferential direction by the proximal slit, and
the at least one separation portion is each connected to a proximal end of a corresponding one of the strut groups.

17. The medical device according to claim 13, wherein
distal parts of the plurality of struts include a plurality of distal struts that extends in the axial direction and is independent from each other in the circumferential direction, and
the distal part of the expansion body includes a distal shaft portion that fixes the plurality of distal struts in such a manner that two adjacent distal struts are not separated from each other in the circumferential direction.
